(19) Europäisches Patentamt
European Patent Office
Office européen des brevets

(11) Publication number: **0 075 029**
**B1**

(12) **EUROPEAN PATENT SPECIFICATION**

(45) Date of publication of patent specification: **29.10.86**

(51) Int. Cl.$^4$: **A 61 K 31/505**

(21) Application number: **82901007.3**

(22) Date of filing: **03.04.82**

(86) International application number:
**PCT/JP82/00101**

(87) International publication number:
**WO 82/03328 14.10.82 Gazette 82/25**

(54) **AGENT FOR TREATING CHRONIC OBLITERATIVE LUNG DISEASE.**

(30) Priority: **03.04.81 JP 50150/81**

(43) Date of publication of application:
**30.03.83 Bulletin 83/13**

(45) Publication of the grant of the patent:
**29.10.86 Bulletin 86/44**

(84) Designated Contracting States:
**FR**

(58) References cited:
**FR-A-2 448 542**
**JP-A-46 001 377**
**JP-A-49 054 396**
**JP-A-54 145 696**

(73) Proprietor: **MOCHIDA PHARMACEUTICAL CO., LTD.**
**7, Yotsuya 1-chome**
**Shinjuku-ku Tokyo 160 (JP)**

(72) Inventor: **OHNISHI, Haruo**
**4-14, Higashifunabashi 6-chome**
**Funabashi-shi Chiba 273 (JP)**
Inventor: **ITOU, Chihiro**
**6-21, Aoyamadai 4-chome**
**Abiko-shi Chiba 270-11 (JP)**
Inventor: **MOCHIDA, Ei**
**5-4, Komagome 2-chome**
**Toshima-ku Tokyo 170 (JP)**
Inventor: **TAIRA, Norio**
**11-32, Kunimi 1-chome**
**Sendai-shi Miyagi 982 (JP)**

(74) Representative: **Bressand, Georges et al**
**c/o CABINET LAVOIX 2 Place d'Estienne d'Orves**
**F-75441 Paris Cedex 09 (FR)**

**EP 0 075 029 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Technical Field

This invention relates to the use of a 5-methyl-7-diethylamino-s-triazolo-(1,5-a)-pyrimidine for the manufacture of a pharmaceutical composition for the treatment of chronic occlusive pulmonary disease.

Background Art

Asthma is characterized by an enhanced reactivity of trachea and bronchi against various stimuli, so that it is defined as a disorder in which widespread constriction of respiratory tract is observed as a symptom [American Thoracic Society: Amer. Rev. Resp. Dis. 92, 513 (1965)]. More specifically, it is assumed that when a person is stimulated by specific allergens such as house dust, hogweed (Ambrosia elatior L.) and shellfish or by non-specific stimulations such as cold air, stimulative gas or bacterial infection, granules are released from mast cells in the broncial mucous membrane or from basophilic leukocytes in the blood, and the released granules in their turns liberate chemical mediators such as histamine and SRS—A which stimulate adenocytes of the bronchi to induce supersecretion or dilation of blood vessels and to incite the increase of permeability finally causing the person to become an edema or it is further assumed that the chemical mediators directly constrict the bronchial smooth muscles and narrow the inside of respiratory tract thereby inducing dyspnea. In view of the above pathological processes, various methods for ameliorating symptoms of asthma have been tried. They include, for example, (1) the inhibition of specific reactions by allergens, i.e., an antigen-antibody reaction between the allergen and the antibody, (2) the inhibition of the liberation of chemical mediators from mast cells, and (3) the dilation of constricted respiratory tract and the acceleration of the removal of increased secretions. Various drugs based on these mechanisms are now used as clinical agents for treating asthma.

Bronchodilators, such as xanthine derivatives and β-stimulants are most widely used. These agents however, have the disadvantage of causing very serious adverse reactions. For example, theophylline, a typical xanthine derivative, is a useful drug as it is highly effective against serious asthmatic attacks and those conditions which show resistance to bronchodilators. However, it is necessary that it is administered with utmost care particularly by intravenous injection because it may induce palpitation, nausea, headache and vertigo and although rarely, induce anxiety of the breast, shock and cardiac arrest causing death. On the other hand, bronchodilators typified by isoproterenol cause inevitable adverse reactions, i.e. the enhancement of heart function and the reduction of blood pressure, owing to its stimulating action or the β-receptor of sympathetic nerve system which is the receptive site for the pharmacological action of these drugs. Hence, the administration of the bronchodilators to old patients is prohibited, particularly when they also suffer from coronary insufficiency, angina pectoris, and arteriosclerosis of a medium or higher degree. For this reason, drugs of this type are unsuitable as systemic agents, and are used mainly as inhalants. Furthermore, since these drugs are to be used only at the time when the asthmatic attack occurs and cannot be used for prophylaxis, they are unable to eliminate the causes which disturb comfortable sleep.

Disclosure of the Invention

The present inventors have made extensive investigations in order to develop a drug which has an excellent therapeutic effect against asthma without any serious adverse reactions. Consequently, it has been found by using experimental models in animals which are now considered to be the most objective methods of evaluating agents for treating asthma that 5-methyl-7-diethylamino-s-triazolo(1,5-a)pyrimidine (commonly known as "Trapidil" which is abbreviated TR hereinbelow) has an ameliorating action on bronchial constriction and stricture which are the fundamental causes of the pathological processes of asthma. This discovery has led to the present invention.

TR is a known compound represented by the following formula (I):

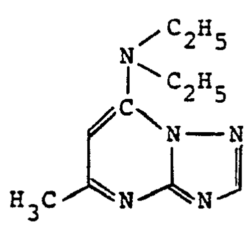

(I)

It is a white to slightly yellowish white crystalline powder with a bitter taste which is readily soluble in water and organic solvents and has been known to have various activities for improving circulatory systems, for example vasodilating activity on the coronary artery [Koroku Hashimoto et al., "Oyo Yakuri" (Pharmacometrics), *8,* 33 (1974)], inhibitory activity on platelet aggregation (Hiroshi Kosuzume et al., "Toho Igakukai Si" (Journal of the Medical Society of Toho University), *26,* 674 (1979)] and an ameliorating activity of lipid metabolism [Haruo Ohnishi et al., "Nichi Yakuri Shi" (Folia Pharmacologica Japonica), *76,* 469 (1980)].

The pharmacological activities of TR will now be illustrated below by experimental examples.

Experimental Example 1

Activities for relaxing smooth muscles of isolated trachea and stimulating isolated atrium:—

Hartley-strain male guinea pigs weighing 400 to 500 g were killed and the trachea and the right atrium were isolated. A preparation of the isolated trachea prepared by the method of Takagi et al. [Takagi, K. et al., Chem. Pharm. Bull., *6,* 716 (1958)] was suspended in Tyrode's solution at 37°C into which air was passed. The preparation of the trachea was made to contract by the addition of histamine ($10^{-6}$M), and thereafter the relaxing effect on the preparation by the addition of the respective test drugs was recorded by a kymographion through a writing lever. On the other hand, a preparation of the isolated atrium was suspended in Krebs-Henseleite's solution at 30°C, while there was passed into this solution oxygen containing 5% carbon dioxide. Increase in heart contractile force and heart rate by the addition of the respective test drugs were recorded by a polygraqph through a strain gauge. The experiments were carried out using three preparations obtained from different animals at each of the concentrations of the drugs, and means values were calculated. The results are shown in Table 1 and Figure 1.

TABLE 1

| | Concentraton (mol) | Percent relaxing ratio of the trachea | Percent increase in heart contractile force | Percent increase in heart rate |
|---|---|---|---|---|
| TR | $1 \times 10^{-5}$ | 18 | 0 | 0 |
| | $3 \times 10^{-5}$ | 93 | 4 | 3 |
| | $1 \times 10^{-4}$ | 200 | 11 | 7 |
| Theophylline | $1 \times 10^{-5}$ | 5 | 0 | 0 |
| | $3 \times 10^{-5}$ | 32 | 4 | 2 |
| | $1 \times 10^{-4}$ | 108 | 11 | 8 |
| Isoproterenol | $1 \times 10^{-9}$ | 0 | 21 | 13 |
| | $1 \times 10^{-8}$ | 0 | 35 | 28 |
| | $1 \times 10^{-7}$ | 123 | 60 | 64 |

The activity of TR in relaxing trachea contracted by histamine was three times a higher that of theopylline, and the activity of TR in enhancing hear function was equivalent to that of theophylline. In other words, TR has three times greater selectivity for trachea as theophylline.

On the other hand, isoproterenol accelerated heart function even at a very low concentration at which it did not show any activity for relaxing trachea.

Experimental Example 2

Bronchodilating action in dog in vivo:—

In the foregoing Experimental Example 1, the inhibitory action of TR for the contraction of an isolated trachea of a guinea pig was described. However, it is desirable in order to determine the utility of TR as an agent for treating asthma by systemic administration, to examine the activity of the drug in vivo.

Thus, the inhibitory activity of TR for the contraction of bronchi in vivo was exmained using dogs.

The experiment was carried out in accordance with the Konzett-Röessler's method [Konzett, H. and Röessler, R: Arch. Exp. Pathol. Pharmacol., *195,* 71 (1940)].

Six mongrel adult dogs were anesthetized with pentobarbital sodium (30 mg/kg). A tracheal cannula was inserted, and under artificial respiration, holes were incised in the right and left pleurae to equalize the pressure of the pleural cavity to the atmospheric pressure. The amount of air which overflowed by inspiration against a pressure of 9.8 ± 0.1 mbar (10 ± 1 cm $H_2O$) applied from the branch of the cannula was measured by a pneumotachograph. The inhibitory action of TR on the increase of the overflowing air due to the intravenous administration of histamine dihydrochloride (2 µg/kg) was taken as an index of the efficacy of TR. The administration of TR was conducted intravenously for two minutes before the administation of histamine dihydrochloride.

The results are shown in Table 2. TR remarkably inhibited the histamine-induced concentration of the bronchi of dogs, and its activity was greater than that of theophylline.

TABLE 2

| Drug | Dose (mg/kg) | Percent inhibition |
|---|---|---|
| Control | | 0 |
| TR | 0.3 | 27 |
| | 1.0 | 48 |
| | 3.0 | 87 |
| Theophylline | 3.0 | 56 |

Experimental Example 3

The selectivity of the dilating activity of TR for trachea was compared with those of isoproterenol and theophylline.

Preparations of blood-perfused trachea in vivo were prepared by the method of Himori and Taira [Himori, N., Taira, N., Brit. Ja. Pharmacol., *56*, 293 (1976)] as follows. None mongrel adult dogs weighing between 10 to 15 kg were anesthetized by the intravenous administration of pentobarbital sodium (30 mg/kg). The cervix of each dog was medisected to expose the pharynx and the upper part of the trachea. A cannula was inserted into the both superior thyroid arteries, and the vascular bed of the trachea was perfused with arterial blood.

The blood was introduced from the femoral artery by a peristaltic pump, and a part of the blood stream was by-passed to the downstream of the femoral artery through a Starling's air resistance tube provided parallel to the vascular bed of the trachea, thereby performing the perfusion under a constant pressure. The pressure of the perfusion was kept a little higher than the mean aortic blood pressure. The blood flow (the amount of blood flow in the vascular bed of the trachea) flowing into the both superior thyroid arteries were measured by an electromagnetic flow meter.

On the other hand, water was placed into the cuff of a tracheal tube equipped with a cuff, and the tube was inserted into the trachea. Then, the inside pressure of the cuff was measured by a pressure transducer to determine the inside pressure of the trachea as follows. Initially, water was placed in the cuff in such a manner that the static inside pressure of the trachea was about 39 mbar (40 cm $H_2O$). During the experiment, the tonus (the inside pressure of the trachea) of the tracheal smooth muscles in stationary phases was maintained by continuous intraarterial injections of 3 μg each of neostigmine into the vascular bed of the trachea at 30-minute intervals.

TR, isoproterenol [(−) isoproterenol hydrochloride] and theophylline were respectively dissolved in 0.9% aqueous solution of sodium chloride, and injected intraarterially by a microsyringe. To the isoproterenol hydrochloride solution was added ascorbic acid in an equimolar amount in order to prevent oxidation.

TR, isoproterenol and theophylline increased the blood flow in the trachea and reduced the inside pressure of the trachea in a dose dependent manner. As an index of the selectivty of the dilating activity for trachea, the ratio of the increase of the blood flow in the trachea to the decrease of the inside pressure of the trachea was determined. The selectivity of TR was about 3 times higher that of isoproterenol. On the other hand, in this experiment, the selectivity of TR was the same as that of theophylline, but the dilating activity for trachea and the increasing activity for tracheal blood flow of TR were about 3 times higher than those of theophylline. Accordingly, TR, unlike sympathomimetic drugs such as isoproterenol, is useful not only for the treatment of asthma but also for the prophylaxis of asthma by oral administration.

Experimental Example 4

Acute toxicity

TR was administered either orally, subcutaneously or intravenously to ICR-strain mice or Wistar-strain rats, and its acute toxicity was examined.

$LD_{50}$ was calculated from the mortality rate according to the method of Litchfield-Wilcoxon three days after the administration.

The results are shown in Table 3. It was proved that TR is a highly safe compound having a high $LD_{50}$ value as compared with the doses which exhibit its efficacy.

TABLE 3

| Animal species | Sex | Oral Administration (mg/kg) | Subcutaneous administration (mg/kg) | Intravenous administration (mg/kg) |
|---|---|---|---|---|
| mouse | male | 740 | 267 | 101 |
| mouse | female | 710 | 253 | 113 |
| rat | male | 750 | 167 | 125 |
| rat | female | 570 | 157 | 113 |

Experimental Example 5 (Clinical Test)

TR was administered to patients with chronic occlusive pulmonary disorders such as bronchial asthma to examine its effect for improving occlusive troubles.

TR was administered orally three times in a dose of 150 mg/day or in some cases 300 mg/day to patients with bronchial asthma, chronic pulmonary emphysema, compliation of these, and chronic bronchitis who had been treated with the drugs shown in Table 4 for several months but had not shown a clear objective or subjective improvement. Before and after the administration, the spirogram and the flow volume curve were recorded to examine the function of the lungs. Furthermore, blood examination and general biochemical tests were performed as required. The results are shown in Table 5.

The improvement of the function of patients with chronic bronchial asthma, etc. by long-term administration should be evaluated carefully because there exists marked time-dependent variation in the condition of the patients including intradaily variation. In order to examine the acute effect of TR, 100 mg of TR was administered intravenously to a healthy young male without a history of respiratory diseases, and forced vital capacity (FVC) was checked before and after the administration (Figure 2 and Table 5). As shown in Table 5, an increase of 0.46 liters in FVC and 0.32 liters in the one second vital capacity ($FEV_{1.0}$) was observed. In the flow volume curve, the peak flow rate (PFR), $V_{.75}$ and $V_{.50}$ showed an apparent increase after the intravenous administration, but $V_{.25}$ and $V_{.10}$ showed a decrease after the intravenous administration. This suggests that TR has a greater dilating effect on larger respiratory tracts.

In cases 1 and 2 with asthma, PFR which is regarded as an index reflecting the seriousness of the occlusion of larger respiratory tracts showed an apparent increase after the administration of TR. This shows that as was noted in the healthy person, TR has improving effects on the functional constriction of larger respiratory tracts.

In cases 4 and 5 mainly with pulmonary emphysema, PFR and $FEV_{1.0}$ decreased although an increase in FVC by 0.44 liters and 0.3 liters was noted. This is presumably due to the aggravation of the occlusionof expiration caused by the relaxing of the bronchial muscles of larger respiratory tracts, i.e. the decrease of the tonus of the tracts, with TR. Occlusive disorders in chronic pulmonary emphysema, unlike bronchial asthma, chronic bronchitis, etc., is caused mainly by the occlusion of expiration due to the decrease in pulmonary elastic contracting pressure, and the tonus of the respiratory tracts itself is related to the occlusion of expiration.

In all cases, the FVC evidently increased after the administration of TR, and an increase in $FEV_{1.0}$ was observed in four cases excepting the cases 4 and 5 of pulmonary emphysema.

Further, TR was administered to a patient with malignant chronic asthma, who had been bothered by continuous wheezing for a year. The thoracic X-ray pattern of the patient indicated that the diameter in the origin of right pulmonary artery was 23 mm and the electrocardiogram showed pulmonary P. This case is described separately from the above cases, because the patient showed severe dypnea, so that the test for pulmonary function could not be performed before the administration of TR.

After the administration of TR in this case, subjective symptoms greatly improved, and a marked decrease in whistling and rattling was objectively noted by ausculation.

In all cases, neither abnormality in the liver function and peripheral blood, nor other adverse effects were observed.

TABLE 4

| Case | Age | Sex | Diagnosis | Dose (mg/ day) | Admini-stration period (days) | Drugs used in combination with TR |
|---|---|---|---|---|---|---|
| 1 | 56 | Male | Bronchial asthma | 150 | 360 | Mucolytic agent for mucus in respiratory tract, anti-inflammatory enzyme preparation, broncho-dilator, steroid |
| 2 | 54 | Female | Bronchial asthma, recurrent nerve paralysis | 150 | 74 | None |
| 3 | 54 | Male | Bronchial asthma, chronic pulmonary emphysema | 300 | 25 | Mucolytic agent for mucus in respiratory tract, anti-inflammatory enzyme preparation |
| 4 | 68 | Male | Chronic pulmonary emphysema | 150 | 112 | Bronchodilator, agent for treating cardiac insufficiency |
| 5 | 65 | Male | Chronic pulmonary emphysema | 150 | 34 | Mucolytic agent for mucus in respiratory tract, anti-angina agent, cardiac agent |
| 6 | 69 | Male | Chronic bronchitis, broncho-dilation | 150 | 82 | Mucolytic agent for mucus in respiratory tract, anti-inflammatory enzyme preparation, bronchodilator |

TABLE 5

| Case | Changes in clinical condition | FVC(l) | | $FEV_{1.0}$(l) | | $FEV_{1.0\%}$(%) | | PFR(l/sec) | | $V_{\cdot 25}$(l/sec) | |
|---|---|---|---|---|---|---|---|---|---|---|---|
| | | Before | After | Before | After | Before | After | Before | After | Before | After |
| 1 | Improved | 2.75 | 2.77 | 1.69 | 1.78 | 61.4 | 64.2 | 4.09 | 4.89 | 0.58 | 0.46 |
| 2 | Improved (an attack of dyspnea in the early morning was alleviated) | 2.44 | 3.58 | 1.70 | 2.72 | 69.8 | 75.9 | 2.69 | 4.05 | 0.59 | 1.31 |
| 3 | Slightly improved (short breath during body movement was alleviated) | 1.37 | 2.37 | 0.44 | 0.77 | 31.7 | 32.4 | 1.67 | 2.50 | 0.12 | 0.11 |
| 4 | No change | 2.64 | 3.08 | 0.71 | 0.60 | 26.9 | 19.4 | 2.49 | 2.12 | 0.21 | 0.16 |
| 5 | Slightly improved (short breath during body movement was alleviated) | 1.09 | 1.39 | 0.42 | 0.42 | 38.2 | 30.2 | 1.43 | 1.06 | 0.13 | 0.05 |
| 6 | Slightly improved | 2.07 | 2.60 | 1.23 | 1.40 | 59.3 | 53.8 | 3.55 | 3.73 | 0.39 | 0.38 |
| Average | | 2.06 ±0.62 | 2.63 ±0.67 | 1.03 ±0.53 | 1.28 ±0.79 | 47.8 ±16.2 | 45.9 ±20.1 | 2.65 ±0.94 | 3.05 ±1.28 | 0.33 ±0.19 | 0.41 ±0.42 |
| | Healthy person | 4.07 | 4.53 | 3.82 | 4.14 | 93.8 | 91.4 | 10.66 | 11.09 | 3.15 | 2.80 |

FVC: forced capacity, $FEV_{1.0\%}$: one second ratio, PFR: peak flow rate, $V_{\cdot 25}$: the flow rate of 25% VC,
$FEV_{1.0}$: one second vital capacity

The above Experimental Examples led to the conclusion that in the experimental models of trachial and bronchial contraction, TR apparently shows an inhibitory activity for the contraction and its action is selective for tracheal muscles suggesting that TR may be used in the treatment of asthma or chronic obstructive pulmonary diseases with bronchial constriction, for example, chronic bronchitis or chronic emphysema. It has been recognized taht from the comparison between effective doses and $LD_{50}$ values in the experimental examples in vivo, the safety of TR is high.

Brief Description of the Drawings

Figure 1 is an example of a graph which shows the contracted state of isolated trachea sample by histamine and the relaxed state of the sample by Trapidil or theophylline in Experimental Example 1.

Figure 2 is a flow volume curve of a healthy young male before and after the administration of Trapidil in Experimental Example 5.

Best Mode for Carrying Out the Invention

It has been found that TR has a dilating activity for trachea and bronchi quite independently of the known pharmacological activities such as its ameliorating activity for circulatory systems e.g. vasodilating activity of coronary arteries, inhibitory activity of platelet aggregation or ameliorating activity of lipid metabolism and therefore it is useful as an agent for the treatment of chronic occlusive pulmonary diseases.

The dose of TR as an agent for the treatment of chronic occlusive pulmonary diseases may be usually 150 to 600 mg (oral agents), 50 to 200 mg (injections) or 10 to 50 mg (inhalant) per day, but may be properly increased or decreased according to the condition, age and sex of patients.

In the clinical use of TR according to this invention, it is formulated in a customary manner into tablets, capsules, aerosols, syrups or injections. It may also be in other forms such as granules and powders. The formulation into these preparation forms can be carried out by conventional methods with acceptable carriers or excipients.

Some examples of the formulations are shown below without any intension of limiting it to these specific examples.

Example 1

Tablets:—

| | | |
|---|---|---|
| (I) | TR | 50 g |
| (II) | Lactose | suitable amount |
| (III) | Crystalline cellulose | 60 g |
| (IV) | Potato starch | 54 g |
| (V) | Magnesium stearate | 2 g |

The ingredients (I) to (IV) were mixed, and a part of the ingredient (IV) previously separated was added as a 10% starch paste, and the mixture was formed into granules. The granules were dried, and the remaining powder which did not form granules was removed by sieving. The ingredient (V) was added to the granules and they were mixed. Tablets of the weight of 200 mg were prepared from mixture by a tableting machine. As required, the tablets may be sugar-coated as usual.

Example 2

Capsules:—

| | | |
|---|---|---|
| (I) | TR | 50 g |
| (II) | Calcium phosphate | 50 g |
| (III) | Aluminium silicate | suitable amount |
| (IV) | Crystalline cellulose | 60 g |
| (V) | Magnesium stearate | 2 g |

The ingredients (I) to (V) were mixed and sieved for further mixing. The mixture was formed into capsules each having a weight of 200 mg in accordance with a conventional method.

Example 3

Aerosols:—

| | | |
|---|---|---|
| (I) | TR | 1.5% |
| (II) | Ascorbic acid | 0.10 |
| (III) | Ethyl alcohol | 35.75 |
| (IV) | Freon® | sufficient amount |

The ingredients (I) and (II) were dissolved in 95% ethyl alcohol (III), and the solution was placed into containers together with the ingredient (IV) in a customary manner.

Example 4

Injections:—

One hundred grams of TR crystals were dissolved in 2 liters of distilled water for injection, and the solution was formed into injections (100 mg/2 ml per ampoule).

**Claims**

1. The use of 5-methyl-7-diethylamino-s-triazolo-(1,5-a)-pyrimidine for the manufacture of a pharmaceutical composition for the treatment of chronic occlusive pulmonary disease.
2. The use according to claim 1 wherein the chronic occlusive pulmonary disease is asthma.
3. The use according to claim 1 wherein the carrier of the pharmaceutical composition is liquid.
4. The use according to claims 1 or 2 wherein the composition is in the form of an oral preparation.
5. The use according to claim 4 wherein the composition is formed into a tablet, capsule, syrup, granule or powder.
6. The use according to claims 1 and 2 wherein the composition is in the form of an injectable solution or an inhalant.

**Patentansprüche**

1. Verwendung von 5-Methyl-7-diethylamino-s-triazolo-(1,5-a)-pyrimidin für die Herstellung einer pharmazeutischen Zusammensetzung zur Behandlung chronischer Lungenverschluß-Erkrankung en.
2. Verwendung nach Anspruch 1, wobei die chronische Lungenverschluß-Erkrankung Asthma ist.
3. Verwendung nach Anspruch 1, wobei der Träger der pharmazeutischen Zusammensetzung flüssig ist.
4. Verwendung nach Ansprüchen 1 oder 2, wobei die Zusammensetzung in Form eines oralen Präparats vorliegt.
5. Verwendung nach Anspruch 4, wobei die Zusammensetzung in die Form von Tabletten, Kapseln, Sirup, Granulat oder Pulver gebracht ist.
6. Verwendung nach Ansprüchen 1 oder 2, wobei die Zusammensetzung in Form einer injizierbaren Lösung oder eines Inhalationsmittels vorliegt.

**Revendications**

1. Utilisation de la 5-méthyl-7-diéthylamino-s-triazolo (1,5-a) pyrimidine pour l'obtention d'une composition pharmaceutique pour le traitement d'une maladie pulmonaire obstructive chronique.
2. Utilisation selon la revendication 1, où la maladie pulmonaire obstructive chronique est l'asthme.
3. Utilisation selon la revendication 1, où le véhicule de la composition pharmaceutique est liquide.
4. Utilisation selon les revendications 1 ou 2 où la composition est sous forme d'une préparation administrable par voie orale.
5. Utilisation selon la revendication 4, où la composition est mise sous forme d'un comprimé, une capsule, un sirop, un granulé ou une poudre.
6. Utilisation selon les revendications 1 et 2, où la composition est sous forme d'une solution injectable ou d'un produit pour inhalation.

FIG. 1

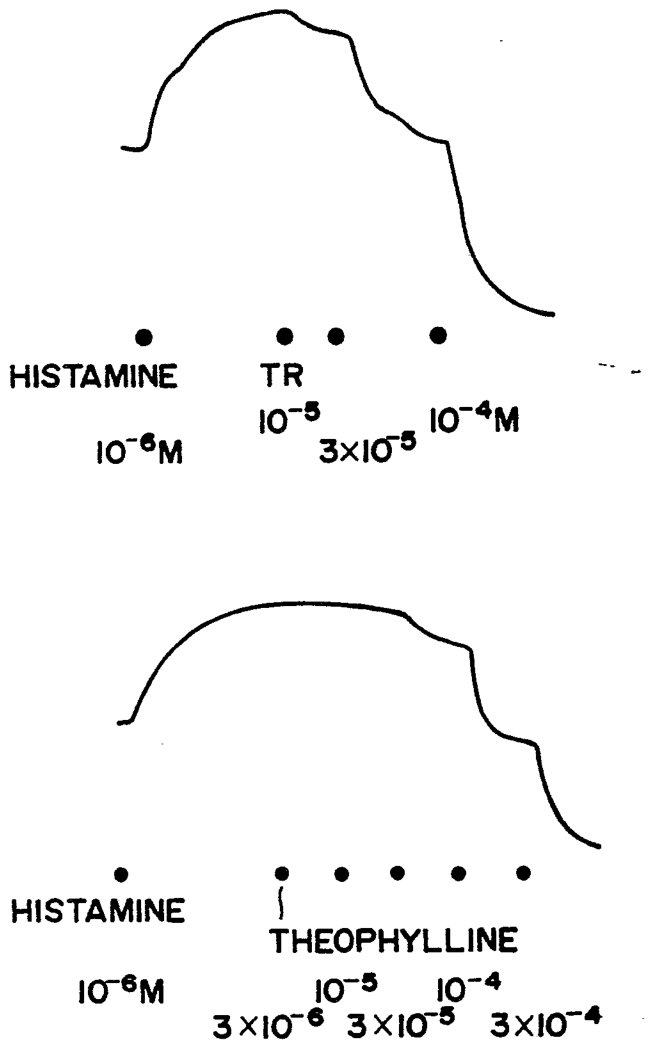
HISTAMINE
TR
10⁻⁶M
$10^{-5}$
$3 \times 10^{-5}$
$10^{-4}$M
HISTAMINE
THEOPHYLLINE
$10^{-6}$M
$3 \times 10^{-6}$
$10^{-5}$
$3 \times 10^{-5}$
$10^{-4}$
$3 \times 10^{-4}$

FIG. 2

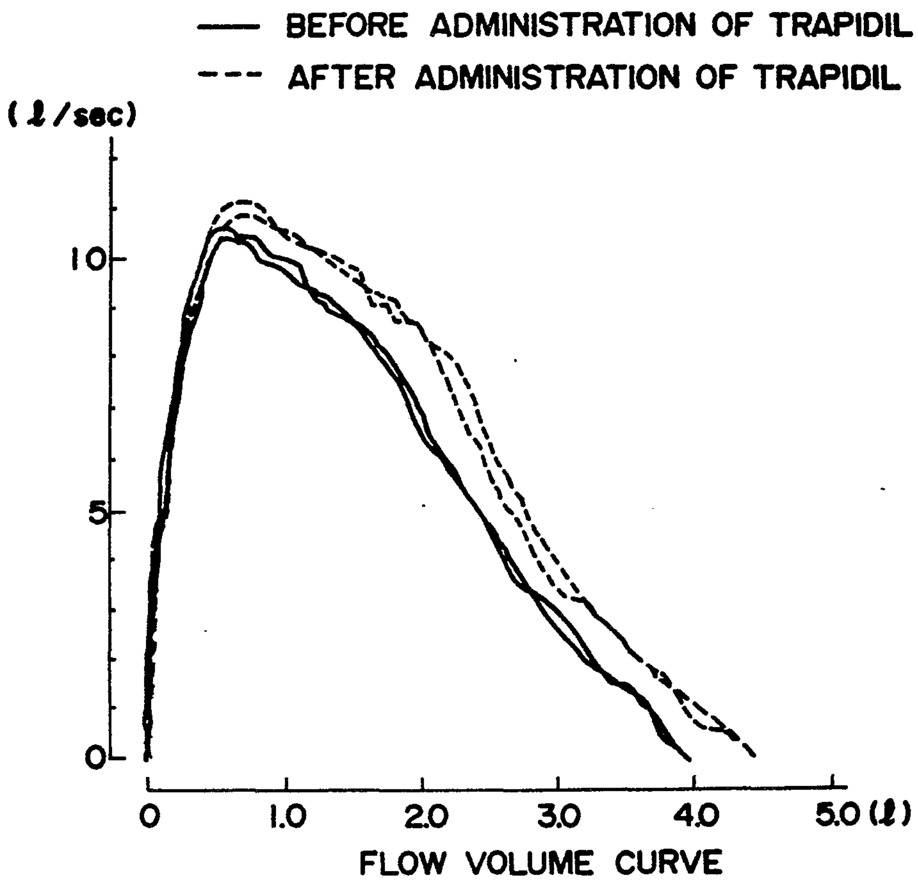
BEFORE ADMINISTRATION OF TRAPIDIL
AFTER ADMINISTRATION OF TRAPIDIL
(ℓ/sec)
10
5
0
0
1.0
2.0
3.0
4.0
5.0 (ℓ)
FLOW VOLUME CURVE